# EUROPÄISCHE PATENTSCHRIFT

(11) **EP 1 909 874 B1**
(45) Veröffentlichungstag und Bekanntmachung des Hinweises auf die Patenterteilung: **27.02.2019**
(21) Anmeldenummer: 06777933.0
(22) Anmeldetag: 24.07.2006
(51) Int. Cl.: A61M 15/00, A61J 1/03, B65D 75/34, B29K 305/02, B29L 31/00, B29C 65/48, B29C 65/00, B29K 23/00, B29K 27/06, B29K 67/00, B29K 69/00, B29K 77/00, B29C 65/50, B29K 27/00, B29K 27/12, B29C 65/02, B65D 75/32

(54) **ARZNEIMITTELBLISTER**
DRUG BLISTERS
BLISTERS POUR MÉDICAMENTS

(30) Priorität: 27.07.2005 DE 102005035705
(43) Veröffentlichungstag der Anmeldung: 16.04.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: METZGER, Burkhard, 55218 Ingelheim am Rhein (DE); GESER, Johannes, 55216 Ingelheim am Rhein (DE); HOELZ, Hubert, 55413 Oberheimbach (DE)
(74) Vertreter: Simon, Elke Anna Maria
(86) Internationale Anmeldenummer: PCT/EP2006/064581
(87) Internationale Veröffentlichungsnummer: WO 2007/012628

(56) Entgegenhaltungen:
- EP-A- 0 905 042
- EP-A1- 0 703 157
- WO-A-00/75037
- WO-A2-2004/041672
- DE-A1- 4 106 379

## Beschreibung

Die vorliegenden Erfindung betrifft Arzneimittelblister für Pulverinhalatoren. Die erfindungsgemäßen Blister beinhalten eine inhalationsfähige Pulverformulierung enthaltend einen Arzneistoff. Die Blister zeichnen sich durch eine reduzierte Permeabilität für Wasserdampf aus.

### Stand der Technik

Die medizinische Aerosoltherapie, bei der ein Wirkstoff inhalativ über die Lunge aufgenommen wird, spielt eine wichtige Rolle in der Behandlung von zahlreichen Lungenkrankheiten. Um die Arzneimittel auszubringen werden häufig Vernebler, Dosieraerosole oder Trockenpulverinhalatoren eingesetzt.

Auf dem Gebiet der Pulverinhalatoren sind Einzeldosen und Mehrdosen-Geräte bekannt. Besonders interessant sind Geräte, bei denen Einzeldosen der Arzneimittelformulierung in verpackten Einheiten vorliegen, besonders in Form von Blisterverpackungen.

Die Art und Weise, wie die Pulverformulierung in dem Gerät verpackt vorliegen, ist für die Produktqualität und damit die Eignung für die inhalative Anwendungen entscheidend.

Die DE 3348370 und die DE 3336486 offenbaren Inhalatoren, die eine scheibenförmige Blisterpackung, aufweisen. Die Blister bestehen aus einer Bodenfolie oder Bodenplatte, die mehrere kreisförmig angeordnete Kavitäten aufweist. Die Kavitäten können nach einer oder nach zwei gegenüber liegenden Seiten hin geöffnet sein. Die Öffnungen befinden sich dementsprechend alle auf der gleichen Stirnseite oder auf beiden Stirnseiten. Die einzelnen Kavitäten enthalten jeweils eine Dosis eines zur Inhalation bestimmten MedikamentenPulvers. Die Öffnungen der Kavitäten sind durch eine Siegelfolie geschlossen. Zum Ausbringen des Medikamentenpulvers wird die Kavität geöffnet. Ein Luftkanal verbindet die geöffnete Kavität mit dem Mundstück des Inhalators. Beispielhaft wird der Inhalator der DE 3336486 näher beschrieben. Dieser weist ein Gehäuse auf, in dem sich eine Kammer (Vorratskammer) findet, die einen Lufteinlass aufweist und in welcher sich ein scheibenförmiger, runder Blister mit abgepackten Medikamententaschen befindet. Der Blister ist über die Stirn- bzw. Bodenseite lose mit der Stirnseite einer runden, rotierbaren Scheibe verbunden. Auf der Scheibe sind umlaufend so Löcher ausgebildet, dass die Medikamententaschen des Blisters über bzw. den Löchern der rotierbaren Scheibe angeordnet werden können. Der Inhalator weist einen Kolben auf, der so angeordnet ist, dass er jeweils eine Medikamententasche durchstoßend öffnen kann, so dass das Medikament in eine Kammer freigesetzt wird und über einen Luftkanal durch das Mundstück vom Benutzer eingeatmet werden kann. Es wird auf die Zeichnungen der Patentanmeldung bzw. der US-Patentschrift verwiesen.

Die DE 4106379 beschreibt ein Inhalationsgerät, in das ein Blisterband eingebracht wird, welches eine Reihe mehrere linear angeordneter Kavitäten mit der pulverförmigen Medikamentformulierung aufweist. Das Blisterband besteht aus zwei voneinander abziehbaren Materialbahnen, die mittels eines vinylischem abziehbarem Wärmeversiegelungslacks miteinander verbunden sind. Das Blisterband weist eine Basisfolie auf, die Kavitäten beinhaltet, wobei die Öffnungen aller Kavitäten auf der gleichen Seite der Basisfolie liegen. Die Öffnungen werden durch die darüber liegende Siegelfolie verschlossen. Das Gerät ist mit einer Einrichtung versehen, die zum Öffnen eines Behälters die beiden Materialbahnen an einer Öffnungsstation voneinander abzieht. Über ein Auslassteil, etwa ein Mundstück, das mit dem geöffneten Behälter verbunden ist, kann der Benutzer das pulverförmige Medikament aus dem geöffneten Behälter inhalieren. An der Öffnungsstation kann eine Antriebseinrichtung vorgesehen sein, die die Trägerbahn und die Abdeckbahn voneinander abzieht. Diese Antriebseinrichtung besteht z.B. aus zwei Antriebsrädern (z.B. Zahnräder), welche die Abdeckbahn in Antriebseingriff zwischen sich halten. Auch in diesem Fall definiert jede einzelne Blisterkavität ein Art Vorratskammer im Inhalator, die über einen Luftkanal mit dem Mundstück verbunden ist.

Die WO00/75037A beschreibt eine Blisterpackung, die ein Bodenteil mit einer Vertiefung enthält, deren Öffnung durch eine auf das Bodenteil aufgesiegelt Abdeckfolie verschlossen ist. Im Bodenteil sind Öffnungshilfen vorgesehen, welche sich entlang von Schwächungslinien umbiegen lassen, so dass mit Kanten oder Spitzen an den umgebogenen Bereichen die über der Vertiefungsöffnung liegende Abdeckfolie derart geschwächt wird, dass sich der Inhaltsstoff von Hand aus der Vertiefung durchdrücken lässt. Das Bodenteil kann ein geprägtes, gegossenes, tief- oder streckgezognes oder vakuumgeformtes Bodenteil aus Metall, wie Aluminium, aus Kunststoff, Kunststoffverbunden wie Kunststoff-Papier-Verbunden oder Kunststoff-Metall-Verbunden sein. Als geeignete Kunststoffe für Bodenteile werden Folien und Folienverbunde angegeben, die Thermoplaste auf Olefin-Basis, Polymere auf Ester-Basis, Polyamide oder halogenhaltige Kunststoffe oder Gemische davon enthalten. Als Beispiel für eine Sperrschicht gegen Gase und Dämpfe im Bodenteil zeigt die WO00/75037A die Einbettung einer in einem Kunststoffverbund eingebetteten Metallfolie auf (z.B. Folienverbund mit PVC und Polyamid und einer Aluminiumfolie dazwischen). Die Abdeckfolie ist beispielsweise eine mit einer Siegelschicht veredelte Aluminiumfolie oder ein aluminiumhaltiger Verbund. Die Siegelschicht besteht beispielsweise aus Heißsiegellack (Dicke 8-50 Mikrometer), Polyolefinen, PP oder PE. Alternativ zum Siegeln wird die Abdeckfolie durch Kleben mit dem Bodenteil verbunden.

Die EP0905042A beschreibt eine Blister- oder Durchdrückpackung mit Bodenteil und Deckel aus metall- und/oder kunststoffhaltigen Folien, wobei in den Bodenteilen eingeformte Vertiefungen am Boden eine Prägung aufweisen. Bei Lyophilisation oder Gefriertrocknung von pharmazeutischen Produkten wird das Produkt als Flüssigkeit in die Vertiefungen eingefüllt und durch Gefrieren und Verdampfen oder Sublimieren bleiben die in der Flüssigkeit enthaltenen Feststoffe als Tablettenform in den Vertiefungen zurück. Die entstehenden Tabletten weisen das Abbild der Prägung vom Boden der Vertiefung auf. Als metall- und/oder kunststoffhaltigen Folien werden Laminate verwendet, die aus einer undurchlässigen Zwischenschicht aus Metall (z.B. Aluminium, Zwischenschicht mit Sperrwirkung gegen den Durchtritt von Wasserdampf, Gasen etc.) und beidseitig der Zwischenschicht angeordneten äußeren Schichten aus kunststoffhaltigen Folien gebildet werden. Als Materialbeispiel für die kunststoffhaltigen Folien werden thermoplatische Kunststoffe (Cycloolefin-Copolymere, Polyolefine, PVC, Polyester und Polyamid) genannt. Die Laminate weisen optional eine außenliegenden siegelfähigen Schicht, z.B. aus Polyethylenen oder Siegellacken, und/oder eine Sperrschicht, z.B. aus thermoplastischen Kunststoffen, auf.

Die WO2004/041672 A2 beschreibt eine Blisterpackung.

Eine der Aufgaben der Blister ist es, die Inhalationsformulierung vor chemischer oder physikalischer Veränderung zu schützen. Zu den physikalischen Veränderungen zählen dabei insbesondere Veränderungen, die das Ausbringen der vorbestimmten Feinpartikeldosis verändern können. Unter dem Begriff Feinpartikeldosis versteht man dabei die Dosis, die die Lunge des Patienten erreicht. Letztere wird von den Wechselwirkungen der mikronisierten Wirkstoffpartikel untereinander als auch der Wechselwirkungen mit den Hilfsstoffen beeinflusst. Es hat sich gezeigt, dass besonders durch Änderung des Feuchtigkeitsgrades im Inneren der Verpackung diese Wechselwirkungen derart zunehmen können, dass die Feinpartikeldosis deutlich vermindert ist. Derartige Veränderung schließen dabei das Eindringen von Wasser in die Verpackung genauso ein, wie das Entfernen von Wasser aus dem Inneren der Verpackung.

Daher ist es eine Hauptaufgaben der Blisterverpackung, die chemische Zusammensetzung der Atmosphäre im Inneren der Verpackung konstant zu halten, um physikalischen oder chemischen Veränderungen der Wirkstoffformulierung vorzubeugen, bzw. die Inhalationsformulierung stabil zu halten. In diesem Zusammenhang unterscheidet man zwischen einer auf eine kurze Zeit ausgerichteten Stabilität, die die Inhalationsformulierung *per se* besitzen muss, auch wenn sich nicht durch das Packmittel ausreichend geschützt wird (*"in-tise-Stabilität"*) und der Langzeitstabilität, d.h. der Stabilität, die gewährleistet sein muss, solange sich die Inhalationsformulierung in dem ungeöffneten Packmittel befindet.

Die Verpackung muss also primär gewährleisten, dass die Inhalationsformulierung langfristig stabil bleibt. Kann das Material und die konstruktive Gestaltung des Primärpackmittels dies nicht gewährleisten, bedarf es des Sekundärpackmittels.
Die Auswahl eines geeigneten Materials für das Primärpackmittel wird durch zwei Faktoren bestimmt: Zum einen muss das Material die angesprochene Schutzfunktion erfüllen können. Zum anderen muss das Material derart sein, dass der Primärpackung die zur Verwendung in dem Pulverinhalator notwendige Form gegeben werden kann und das Primärpackmittel die ihr zugedachte Funktion erfüllen kann.

Um die Wasserdampfdurchlässigkeit der Blister zu vermindern, wird im Stand der Technik das Augenmerk besonders auf die Folienmaterialien gerichtet, aus denen die Blister aufgebaut sind. Zu den erfindungsgemäß interessanten Blistern zählen Blister mit einer Bodenfolie aus Kunststoff und/oder Aluminium und einer Siegelfolie aus Aluminium. Dabei wird die Siegelfolie über einen Siegellack, der auf die gesamte Oberfläche der Siegelfolie aufgetragen wird, mit der Bodenfolie verklebt.

Überraschend wurde nun gefunden, dass bei den beschriebenen Blistern nicht nur die Folienmaterialien für eine Permeation von Wasserdampf von Außen in den Blister verantwortlich sind, sondern unerwarteter Weise die Siegellackschicht, mit der die Siegelfolie auf die Bodenfolie fixiert wird, einen signifikanten Beitrag leistet. Der Befund erstaunt insbesondere deshalb, weil die Höhe der Siegellackschicht nur wenige Mikrometer beträgt und damit kaum Anteil an der Oberfläche des Blisters hat.

### Beschreibung der Erfindung

Die vorliegende Erfindung betrifft Blister für Arzneimittel für Ein- oder Mehrdosispulverinhalatoren. Bevorzugt sind Blister, bei denen mehrere Einzeldosen der Inhalationsformulierung in einem Blisterband oder einer Blisterscheibe verpackt vorliegen. Die Blister zeichnen sich dabei durch die Höhe der Siegellackschicht, bzw. der bei einem versiegelten Blister nach Außen hin sichtbaren Oberfläche der Siegellackschicht aus.

Erfindungsgemäß ist es von untergeordneter Bedeutung, ob in den Blistern die Inhalationsformulierung direkt verpackt vorliegen (Blister ist Primärverpackung) oder ob die Inhalationsformulierung in einem weiteren Behälter verpackt vorliegt (Blister ist schützende Sekundärverpackung).
Erfindungsgemäß sind Blister bevorzugt, die als Primärverpackung ausgebildet sind und Bestandteil eines gebrauchsfertigen Pulverinhalators sind.

Das Merkmal "Bestandteil" oder "integraler Bestandteil eines einsatzfähigen oder gebrauchsfertigen Inhalators" bedeutet, dass der Blister ein Element in dem Inhalator ist, ohne das die Beschickung des Inhalators mit der Arzneimittelformulierung (Inhalationsformulierung) zum Zweck der Inhalation nicht möglich ist bzw. nicht vorgesehen ist. Dieses Element kann in gebrauchsfertigem Zustand (einsatzfähigen Zustand) fest mit dem Inhalator verbunden sein, so dass es nicht zerstörungsfrei oder ohne den Inhalator zu beschädigen entnommen werden kann, oder es ist zerstörungsfrei lose bzw. lösbar mit dem Inhalator verbunden.

Einsatzfähig bedeutet, dass der erfindungsgemäße Blister in den Inhalator eingesetzt vorliegt.

Die erfindungsgemäßen Blister sind besonders zur Verpackung von wasserempfindlichen Inhalationspulvern geeignet.

Die Aufgabe der Blisterverpackung besteht darin, den Austausch von Materie zwischen ihrem Inneren und der Umgebung zu minimieren.

Eine weitere Aufgabe der Erfindung besteht darin, Inhalationsformulierungen mit einem wasserempfindlichen Arzneistoff in einem Mehrdosis-Pulverinhalator mit vordosierten Einzeldosen so zu verpacken, dass das Eindringen von Feuchtigkeit in die Formulierung gegenüber den aus dem Stand der Technik bekannten Behältnissen verzögert wird.

Eine weitere Aufgabe besteht darin, den Durchtritt von Wasserdampf durch die Siegellackschicht gegenüber den aus dem stand der Technik bekannten Systemen zu reduzieren.

### Beschreibung der Erfindung im Detail

Die vorliegende Erfindung betrifft Blister mit einzelnen oder aneinander gereihten Blistertaschen oder Kavitäten, insbesondere Blisterbänder oder Blisterscheiben. Die Blister bestehen aus einer Bodenfolie mit Kavitäten und wenigstens einer Siegelfolie. Die beiden Folien sind über eine Siegellackschicht miteinander verbunden. Die Siegellackschicht wird meist flächendeckend auf einer der beiden Stirnseiten der Siegelfolie aufgebracht. Diese Seite der Siegelfolie wird dann unter Wärme und/oder Druck flächendeckend mit der Seite der Bodenfolie verbunden, auf der die Öffnungen der Kavitäten ausgebildet sind. Gegebenenfalls wird die Bodenfolie von beiden Seiten derartig versiegelt.

Die Gestalt des erfindungsgemäßen Blisters wird ggf. durch den zu verwendenden Pulverinhalator vorgegeben.

Erfindungsgemäß handelt es sich bei den Blistern demnach um eine Verbundfolie bzw. ein Laminat aus
a) einem Basiselement mit wenigstens einer nach wenigstens einer Seite hin offenen Kavität,
b) wenigstens einer Siegelfolie, die mit dem Basiselement so verschweißt wird, dass die wenigstens eine Kavität unter Ausbildung eines Holraums mit einer darin eingebetteten Arzneimittelformulierung verschlossen wird und
c) einer dazwischen liegende Siegellackschicht, die die das Basiselement und die Siegelfolie miteinander verbindet.

Im einfachsten Fall besteht demnach der Blisters aus drei Schichten: unten befindet sich das Basiselement mit der nach oben hin offenen Kavität, darüber die Siegellackschicht und darüber die Siegelfolie. Auf beiden Seiten dieses Laminats können weitere Schichten ausgebildet sein. Die Art der verwendeten Materialien und die Anzahl der Schichten beeinflusst die Wasserdampfpermeabilität von außen in die Kavität und *vice versa* sowie die mechanische Stabilität des Blisters.

### Materialien

Das Basiselement wird von einer Metallfolie, Polymerfolie, einem Metall- und/oder Polymerkörper gebildet. Als Metall oder Metallfolie wird Aluminium bzw. eine Aluminiumfolie oder eine Aluminiumverbundfolie aus Aluminium und z.B. einem Kunststoff bevorzugt. Als Material für die Kunststofffolien kann PVC (Polyvinylchlorid), COC (Cycloolefin- Copolymer, z.B. Topas®), Cycloolefinpolymer (COP), Polychlorotrifluorethylen (z.B. ACLAR®), Polyethylen (z.B. als high density Polyethylen oder low density Polyethylen), Polypropylen, Polyvinylidenchloride (PVDC), Polyethylenterephthalalt, Polycarbonate, Polyester, Polyacrylate, Polyamide oder ein anderer Kunststoff verwendet werden.

Das Basiselement ist häufig eine Tiefzieh-Folie, in der die Kavitäten ausgebildet sind. Derartige Tiefziehfolien werden im vorliegenden Kontext auch Wannenfolien genannt, wenn in der Folie Wannen oder Näpfe zur Aufnahme der pharmazeutischen Formulierung ausgebildet sind.

Alternativ kann das Basiselement auch eine feste Kunststoff- oder Metallscheibe oder - fläche sein.

In einer weiteren anderen Ausführungsform besteht das Basiselement aus einem Kunststoff oder einer Kunststofffolie, wobei das Basiselement vollständig oder partiell mit einer Metallfolie verkleidet ist, z.B. Ausführungsformen, bei denen zumindest die Kavität (Kavitäten) vollständig mit der Metallfolie ausgekleidet sind.
Der innere Teil der Wandung ist dabei der Teil der Wandung der Medikamententasche, die mit der Arzneimittelformulierung in Berührung kommen kann.

Bevorzugt besteht das Basiselement aus einer Aluminiumfolie, die ggf. auf der dem Produkt zugewandten Seite (Seite mit der Kavität) eine Kunststoffbeschichtung aufweisen kann. In diesem Fall ist die Kunststfoffbeschichtung möglichst dünn, ihre Höhe ist bevorzugt kleiner gleich 50 Mikrometer, bevorzugt kleiner gleich 40 Mikrometer, bevorzugt kleiner gleich 30 Mikrometer.

Die Siegelfolie ist eine Metall- und/oder Polymerfolie. Sie kann aus den gleichen Materialien wie das Basiselement gefertigt sein.

Als Siegellack kann ein Heißlack verwendet werden. Geeignet sind Heißsiegellacke auf Basis eines Polyacrylats und/oder Polyethylens (z.B. high density und/oder low density Polyethylen). Bevorzugt sind Materialien mit geringen Permeationskoeffizienten für Wasser. Bei Gemischen wird erfindungsgemäß der Anteil der Bestandteile mit hoher Permeation soweit möglich gesenkt. Häufig finden Gemische aus Polymethylacrylat und Polyethylacrylat Verwendung. Bevorzugt sind Siegellacke, die ein Gemisch aus Polymethylacrylat und Polyethylacrylat enthalten, bei denen der Anteil an Polymethylacrylat zu Polyethylacrylat 50 bis 100 % Gew. Beträgt.

Ergänzende Schichten können aus den oben genannten Materialien oder Papier bestehen.

Ein bevorzugter Blister besteht aus einem Basiselement aus einer Aluminium- oder Aluminiumverbundfolie und einer Siegelfolie aus einer Aluminium- oder Aluminiumverbundfolie. Dazwischen liegt dann die erfindungsgemäße Siegellackschicht.

### Ausgestaltung des Basiselements

Das Basiselement stellt eine Fläche dar, in der Kavitäten ausgebildet sind. Die beiden Seiten der Flächen sind bevorzugt plan, sieht man von den Kavitäten ab.

Bei einem Basiselement mit mehreren Kavitäten sind diese in Reihe, parallel, kreisförmig, spiralförmig oder in mehreren Reihen angeordnet.
Dabei befinden sich die Öffnungen der Kavitäten bevorzugt auf der gleichen Seite des Basiselements.

Bei einem Basiselement mit mehreren Kavitäten mit jeweils zwei Öffnungen sind die beiden Öffnungen bevorzugt gegenüberliegend ausgebildet (*en face*).

Obwohl die Dimensionen der Kavitäten erfindungsgemäß von untergeordneter Bedeutung sind, soll zur Veranschaulichung darauf eingegangen werden.
Im Fall der bevorzugten als Primärverpackungen ausgebildeten Blister beträgt die Länge der Kavität(en) in dem Basiselement bis zu 10 mm, die Breite bis 10 mm und die Höhe bis zu 5 mm bevorzugt bis zu 3 mm. Oft sind die Dimensionen der Kavitäten im Fall der Blister für Pulverinhalatoren geringer, beispielsweise 6,4 mm x 3,7 mm x 1,6 mm (Länge x Breite x Höhe) oder 4 mm x 2,65 mm x 0,9 mm.

Im Fall von Sekundärverpackungen können die Dimensionen dagegen größer sein: beispielsweise 40 mm x 25 mm x 15 mm (Länge x Breite x Höhe).

Jede Öffnung einer Kavität ist von einem senkrecht zur Öffnung orientierten, im Wesentlichen flachen Steg umgeben. Dieser hat eine Breite von wenigstens 0,5 mm, bevorzugt 1 mm, stärker bevorzugt 2 und am stärksten bevorzugt 4 mm.

In den Kavitäten kann die Wirkstoffformulierung unmittelbar vorliegen oder in der Kavität befindet sich zusätzlich eine Wanne (Napf), in der die Wirkstoffformulierung vorliegt. Gegebenenfalls kann der Wirkstoff in den Kavitäten auch verpackt vorliegen, z.B. in Kapseln.

Das Basiselement bzw. der fertige Blister kann als Streifen, Band oder Scheibe ausgebildet sein.

### Fixierung der Siegelfolie und des Basiselements

Die Siegelfolie ist so mit dem Basiselement verbunden, dass die Öffnung jeder Kavität vollständig verschlossen ist, d.h. eine geschlossene Siegelnaht umläuft die Öffnung einer jeden Kavität. Dafür sind die beiden Schichten wenigstens am Rand des Blisters miteinander verschweißt oder verklebt, bevorzugt auch unmittelbar um die Kavität herum. Die Siegelfolie wird bevorzugt überall dort, wo sie das Basiselement berührt durch die Heißlackschicht fest an das Basiselement fixiert (homogene Verteilung des Siegellacks). Im Idealfall klebt die Siegelfolie über ihrer gesamten Fläche auf dem Basiselement mit Ausnahme der Bereiche der Siegelfolie, die sich über den Öffnungen der Kavitäten befinden.

Zum Verbinden der Siegelfolie mit dem Basiselement wird auf die Siegelfolie oder auf das Basiselement eine Siegellackschicht (Verbindungsschicht) bevorzugt homogen aufgetragen, z.B. in Form einer Heißsiegellackschicht. Dann werden die Siegelfolie und das Basiselement so miteinander in Verbindung gebracht, dass sich zwischen den beiden Schichten die Verbindungsschicht befindet. Unter Druck und Wärmezufuhr wird dann bewirkt, dass Verbindungsschicht die beiden anderen Schichten aneinander fixiert. Dabei kann die Verbindungsschicht aufschmelzen. Die Oberflächenstruktur der Siegellackschicht nach dem Versiegelungsvorgang wird u.a. auch durch die Versiegelungswerkzeuge bestimmt. Dabei können die Schichtdicken der Siegellackschicht zwischen zwei benachbarten Punkten voneinander abweichen. Z.B. kann sich die Siegellackschicht im Querschnitt als punktartiges Netzwerk darstellen. Daher umfasst die Bezeichnung "Schichtdicke" im Kontext mit der vorliegenden Erfindungsbeschreibung nicht nur Ausführungsformen mit einer homogenen Schichtdicke und Materialverteilung, sondern auch inhomogene Schichtdicken und Materialverteilungen, wie sie dem Stand der Technik bekannt sind. Der Begriff Schichtdicke gibt dann die entsprechenden Durchschnittswerte wieder. Bevorzugt sind Siegellackschichten mit homogener Verteilung des Lacks.

Bevorzugt sind Blister, bei denen die Öffnung der Kavität auf einer Fläche von mindestens 0,5 mm, bevorzugt 1 mm, stärker bevorzugt 2 und am stärksten bevorzugt 4 mm Breite über den gesamte Umfangs der Öffnung versiegelt ist.

Um die erfindungsgemäße Aufgabe zu lösen, die Wasserdampfpermeabilität der Siegellackschicht zu reduzieren, wird ein Blister vorgeschlagen, bei dem die Höhe der Siegellackschicht nach dem Verschweißungs- bzw. Verklebungsvorgang bis zu 5 Mikrometer beträgt. Besonders bevorzugt sind Querschnittshöhen von 1 bis 4,5 Mikrometer, insbesondere von 2 bis 4,5 Mikrometer. Am Bevorzugtesten sind Querschnittshöhen von 2 bis 4 Mikrometer. Zum Vergleich: Bei einem durchschnittlichen Blister liegt die Gesamthöhe zusammengesetzt aus der Höhe des Basiselements und der Siegelfolie in einer Größenordnung von 80 Mikrometern. Die Höhe ist die Ausmessung der Schweißnaht, die Senkrecht auf der Ebene des Basiselements bzw. der Siegelfolie steht.

Um diese Querschnittshöhe im fertigen Blister zu verwirklichen, kann die Menge an Siegellack vor dem Versiegelungsvorgang bis zu 10 g / Quadratmeter betragen. Bevorzugt sind Mengen von 1 bis zu 7 g/ Quadratmeter, besonders bevorzugt sind 2 bis 5,5 g / Quadratmeter.

Anstelle der absoluten Höhe der Siegellackschicht, kann näherungsweise auch die relative Oberfläche der Siegellackschicht als charakteristisches Merkmal herangezogen werden. Die relative Oberfläche ist die nach Außen hin orientiert Oberfläche und damit die Querschnittsfläche über die Wasserdampf in das System eindringen kann. Die relative Oberfläche beträgt erfindungsgemäß bis zu 0,035 % der Gesamtoberfläche des Blisters. Bevorzugte Werte sind: bis zu 0,03%, stärker bevorzugt bis zu 0,025%, stärker bevorzugt bis zu 0,02%, stärker bevorzugt bis zu 0,015%.

Unter dem Begriff "nach außen hin sichtbare Oberfläche" soll die nach Außen hin orientierte Oberfläche der Nahtstelle verstanden werden. Das ist der Teil der Oberfläche der Nahtstelle der mit der Umgebung des Blisters, d.h. Luft, Wasserdampf, in unmittelbarem Kontakt steht bzw. die Permeationsoberfläche.

Der Begriff "relative Oberfläche" definiert sich durch das Produkt aus der nach außen hin sichtbaren Oberfläche und dem Quotienten aus Kantenlänge der Nahtebene der Siegellackschicht und tatsächlichen Länge der Naht der Siegellackschicht, jeweils bezogen auf eine Blistereinheit aus einer Medikamententasche.

Die tatsächliche Länge der Naht der Siegellackschicht ist die zur Siegelfolie oder dem Basiselement parallel liegende Komponente der nach außen hin sichtbaren Oberfläche.

Die Nahtebene ist eine durch die Nahtstelle aufgespannte, gedachte Fläche, die die Siegelfolie von dem Basiselement trennt. Diese imaginäre Nahtebene soll mit der Siegelfolie abschließen. Deren Kanten liegen naturgemäß in dieser Ebene. Bei Blistern, bei denen einzelne Medikamententaschen nebeneinander angeordnet sind, wird die Kante der Nahtebene nicht vorrangig durch den Abschluss der Siegelfolie definiert, sondern durch den imaginären, mittigen Grenzverlauf zwischen den Medikamententaschen, d.h. Kavitäten.

Liegt zwischen der Kante der Siegelfolie und der Medikamententasche keine weitere Medikamententasche ist hier die Kante der Siegelfolie heranzuziehen oder vorrangig von einem imaginären mittigen Grenzverlauf geschnitten wird.
Durch dieses Vorgehen wird die Nahtebene in Bezug auf jede einzelne Kavität (Medikamententasche) genormt, bzw. jeder Blister wird so behandelt, als hätte er nur eine einzige Medikamententasche. Das gleiche Prinzip wird zur Bestimmung der Länge der Naht der Siegellackschicht angewandt.

Stellt man sich einen quadratischen Blister vor, bestehend aus einer quadratischen Siegelfolie und einem dazu passgenauen Basiselement ergibt sich folgendes Ergebnis: Die Länge der Naht der Siegellackschicht ist gleich vier mal die Kantenlänge des Quadrats.

Die Kantenlänge der Nahtebene ist ebenfalls gleich vier mal die Kantenlänge des Quadrats.

Damit ist die relativ nach außen hin sichtbare Oberfläche der Siegellackschicht identisch mit der absoluten nach außen hin sichtbare Oberfläche der Siegellackschicht.

Stellt man sich den Blister als quadratischen Blister vor, dessen Basiselement und Siegelfolie aus der gleichen Folie besteht, die zunächst mittig gefalzt wurde, dann die beiden Teilelemente übereinander geklappt wurden, ergibt sich ein quadratischer Blister, der auf drei Kantenseiten eine Siegeloberfläche aufweist, während die vierte Seite die Falzseite ist.

In diesem Fall ist die Länge der Naht der Siegellackschicht gleich drei mal die Kantenlänge des Quadrats, während die Kantenlänge der Nahtebene gleich vier mal die Kantenlänge des Quadrats ist. Damit errechnet sich die relative, nach außen hin sichtbare Oberfläche zu 4/3 mal der absoluten Oberfläche.

Bei einem Blister aus zwei quadratischen Einzelblistern, die über eine Kante miteinander verbunden sind, liegt der analoge Fall zu dem oben beschriebene Blister mit drei Siegelkanten vor.

Die Relativierung der Oberfläche führt dazu, dass als Bezugspunkt für die Oberfläche immer die Oberfläche verstanden wird, die sich für einen Blister mit einer Medikamententasche ergibt, bei dem die gesamte Kantenlänge der Nahtebene als Siegelfläche ausgebildet ist.

In einer alternativen Beschreibungsweise lässt sich die Erfindung auch über die Wasserdampfpermeation durch die Siegellackschicht beschreiben. Idealerweise liegt die Permeation im stationären Gleichgewicht ("steady state") bei einer Luftfeuchtigkeit im Raum in der Kavität von ca. 16% und einer Luftfeuchtigkeit in der Außenumgebung des Blisters von 75% und bei einer Temperatur von 40°C über einen Zeitraum von 6 Monaten bei kleiner gleich 320 Mikrogramm / mm² Siegellackschicht, bevorzugt bei 60 bis 280 Mikrogramm / mm² Siegellackschicht, stärker bevorzugt bei 120 bis 280 Mikrogramm / mm² Siegellackschicht, stärker bevorzugt bei 120 bis 250 Mikrogramm / mm² Siegellackschicht.

Unter einem stationäres Gleichgewichtszustand wird der Zustand verstanden, bei dem die Siegellackschicht mit Wasser/Wasserdampf gesättigt ist.

### Besondere Ausgestaltungsformen

Eine Ausführungsform des erfindungsgemäßen Blister betrifft eine Blisterscheibe.

Diese besteht aus einer zylinderartigen Scheibe bzw. Bodenplatte, die bevorzugt eine Höhe von bis zu 5 mm Höhe und einem Durchmesser von bis zu 15 cm aufweist. In der Scheibe sind senkrecht zur Scheibenebene Mulden oder Löcher ausgebildet (Kavitäten). Die Mulden oder Löcher sind bevorzugt am äußeren Rand der Scheibe ausgebildet und können durch eine oder mehrere Siegelfolien verschlossen sein. In den Mulden oder Löchern befindet sich die Inhalationsformulierung. Der Scheibenkörper besteht dabei aus dem erfindungsgemäß eingesetzten Material. Eine derartige Scheibe kann beispielsweise in einem Inhalator gemäß der DE 3348370 oder der DE 3336486 eingesetzt werden. Ein solcher Inhalator weist ein Gehäuse auf, in dem sich der scheibenförmige, runde Blister mit abgepackten Medikamententaschen befindet. Der Inhalator weist u.a. einen Stift auf, der so angeordnet ist, dass er jeweils eine Medikamententasche öffnen kann, so dass das Medikament in die Kammer freigesetzt wird und über ein Mundstück eingeatmet werden kann.

In einer anderen Ausführungsform ist der Behälter ein streifenförmiger flexibler Blister mit einer Reihe von mehreren linear angeordneten Medikamentaschen, wie er in der DE 4106379 beschrieben wird. Der Blister besteht aus wenigstens zwei voneinander abziehbaren Materialbahnen, die wenigstens einen Behälter definieren, in dem sich das Medikament befindet.

Der dazugehörige Inhalator ist versehen mit einer Einrichtung, die zum Öffnen dieses Blisters die beiden Materialbahnen an einer Öffnungsstation voneinander abzieht. Über ein Auslassteil, etwa ein Mundstück, das mit dem geöffneten Behälter verbunden ist, kann der Benutzer das pulverförmige Medikament aus dem geöffneten Behälter inhalieren. Dabei kann eine der Materialbahnen auch eine Trägerbahn mit mehreren Taschen sein und die andere Materialbahn eine Abdeckbahn. Jede Tasche und der angrenzende Bereich der Abdeckbahn bilden dann einen Behälter. An der Öffnungsstation kann eine Antriebseinrichtung vorgesehen sein, die die Trägerbahn und die Abdeckbahn voneinander abzieht. Diese Antriebseinrichtung besteht z.B. aus zwei Antriebsrädern (z.B. Zahnräder), welche die Abdeckbahn in Antriebseingriff zwischen sich halten. Auch in diesem Fall definiert jeder einzelne Blister ein Art Vorratskammer im Inhalator, die über einen Luftkanal mit dem Mundstück verbunden ist.

In dem erfindungsgemäßen Blister sind die nach Außen orientierten Kanten des Blisters umgefalzt und ggf. ist die Umfalzkante mit dem nach Innen liegenden Bereich des Blisters verklebt oder verschweißt. Bevorzugt wird die Umfalzkante vollständig auf das Basiselement oder die Siegelfolie zurückgeklappt. Die Falzkante kann bis zu 4 mm, bevorzugt bis zu 2 mm, stärker bevorzugt bis zu 1 mm entfernt von der Kante liegen. Die Falzung bewirkt zweierlei: Zum einen wird der Permeationsweg für Wasserdampf verlängert, da der Abstand zwischen Kavität und Kante verlängert werden kann, zum anderen stellt die Falzkante *per se* eine Verengung und damit eine weitere Permeationsbarriere dar. Der bevorzugte Abstand zwischen dem Rand der Kavität und der am nächsten liegenden Kante beträgt wenigstens 1 mm, bevorzugt wenigstens 2 mm, stärker bevorzugt wenigstens 3 mm. Wie bereits ausgeführt, kann durch Umfalzung der Abstand ggf. verlängert werden.

Alternativ oder ergänzend dazu kann die Oberfläche jeder Außenkante zusätzlich mit einer weiteren Siegelfolie bedeckt werden (senkrecht zu den Laminatschichten). Die weitere Siegelfolie kann auf die Außenkante beschränkt sein, sie kann auf die oberste Schicht und die untersten Schicht des Blisters übergreifen oder sie kann im Fall einer Umfalzkante die Außenkante überdecken. Die weitere Siegelfolie kann aus dem gleichen Material wie die erste Siegelfolie sein, z.B. eine Aluminiumfolie.

In einer bevorzugten Ausführungsform besteht das Basiselement aus einer Aluminiumfolie und die Siegelfolie ebenfalls. Dazwischen liegt die Siegellackschicht.

In einer weiteren bevorzugten Ausführungsform besteht das Basiselement aus einer Aluminiumfolie, welche mit einer Kunststoffschicht überzogen ist. Darüber liegt die Siegellackschicht und darüber eine Siegelfolie aus Aluminium. In diesem oder ähnlichen Fällen wird die Kunststoffschicht ebenfalls möglichst dünn gehalten. Die Kunststoffschicht ist über eine Klebeschicht mit der Aluminiumfolie des Basiselements verbunden, wobei die Klebeschicht idealerweise dünner als die Siegellackschicht ist, bevorzugt weniger als die Hälfte der Dicke der Siegellackschicht, mindestens weniger als 2,5 Mikrometer. Die Kunststoffschicht hat bevorzugt eine Dicke von kleiner gleich 50 Mikrometer, bevorzugt kleiner gleich 40 Mikrometer, bevorzugt kleiner gleich 30 Mikrometer. Als Kunststoff kann einer der bereits im Zusammenhang mit dem Basiselement erwähnten Kunststoffe verwendet werden, d.h. PVC (Polyvinylchlorid), COC (Cycloolefin- Copolymer, z.B. Topas®), Cycloolefinpolymer (COP), Polychlorotrifluorethylen (z.B. ACLAR®), Polyethylen (z.B. als high density Polyethylen oder low density Polyethylen), Polypropylen, Polyvinylidenchloride (PVDC), Polyethylenterephthalalt, Polycarbonate, Polyester, Polyacrylate, Polyamide, oder andere Kunststoffe. Bevorzugt ist PVC.

Der Schichtaufbau des besonders bevorzugten Aluminiumverbund-Blisters ist wie folgt: Die außenliegende Schicht der Bodenfolie besteht aus Polyamid, welches beispielsweise biaxial gereckt ist. Die nachfolgende Aluminiumschicht fungiert als Wasserdampfbarriere. Sie ist mit der Polyamidschicht verbunden, beispielsweise über einen Kleber/Haftvermittler. Bei dem eingesetzten Aluminium handelt es sich um eine Legierung, die eine gute Kaltverformbarkeit aufweist, beispielsweise die Legierung AA 8021B. Mittels eines Klebers ist die dem Produkt zugewandte Kunststoffschicht mit der Aluminiumschicht verbunden. Die Kunststoffschicht aus PVC hat eine Dicke, wie im obigen Absatz beschrieben. Die Deckfolie besteht aus einer Aluminiumschicht, auf die eine Schicht aus Heißsiegellack aufgebracht ist (beispielsweise eine 4 bis 9 µm dicke Schicht). Die Aufbringung kann im Walzenauftragungsverfahren erfolgen. Beim Aufsiegeln der Deckfolie unter Hitze und Druck auf die verformte Bodenfolie schmilzt der Heißsiegellack und verklebt mit der Kunststoffschicht der Bodenfolie. Auf der Außenseite der Aluminiumschicht ist im Allgemeinen ein Druckvorlack aufgetragen, auf den die Produktbezeichnung, der Hersteller, die variablen Daten etc. aufgedruckt werden. Um diesen Aufdruck vor Abrieb zu schützen, kann noch ein Schutzlack aufgebracht werden.

In einer weiteren Ausführungsform kann jede der bisher beschriebenen Ausführungsformen so weiterentwickelt werden, dass zumindest die äußerste Naht durch zwei Aluminiumfolien und die Siegellackschicht gebildet wird. Zwischen diesen Aluminiumschichten können weitere Schichten, z.B. Kunststoffschichten ausgebildet sein (eingeschlossene Schichten). In diesem Fall sind jedoch die Ausmessungen (Länge und Breite) der eingeschlossenen Schichten etwas kleiner gehalten als die der beiden genannten Aluminiumschichten. Dadurch wird erreicht, dass die Außenkante des Blisters durch einen Steg gebildet wird, der aus den beiden durch die Siegellackschicht zusammengeklebten Aluminiumfolien gebildet wird, ohne dass eine der eingeschlossenen Schichten unmittelbar mit der Außenkante abschließt. Auch hierdurch wird eine minimale Oberfläche für die Permeation für Wasserdampf gebildet. Beispielsweise kann eine Wannenfolie aus Kunststoff auf diese Weise vollständig von einer bodenseitigen und einer deckseitigen Aluminiumfolie umgeben werden, so dass auch die Kante der Kunststoffwanne von den Aluminiumfolien umgeben ist.

### Bevorzugte Arzneimittelformulierungen

Bevorzugte Wirkstoffklassen, die für eine Inhalationsformulierung bereit gestellt werden, die in dem erfindungsgemäßen Behältnis für Pulverinhalatoren vorliegen sind: Anticholinergika, Antimuskarinika, Steroide, Betamimetika, Steroide, PDE-IV-Inhibitoren, LTD4-Antagonisten und EGFR-Hemmer. Besonders bevorzugt sind Anticholinergika, insbesondere Tiotropium. Letzteres liegt ganz besonders bevorzugt als Tiotropiumbromid-Monohydrat vor. Daher betrifft ein Aspekt der Erfindung den erfindungsgemäßen Blister enthaltend Tiotropiumbromid-Monohydrat.

Die in die erfindungsgemäßen Blistern eingesetzten Inhalationspulver enthalten neben dem Wirkstoff bevorzugt wenigstens einen Hilfsstoff. Dieser kann aus einer bezüglich der mittleren Teilchengröße der Hilfsstoffpartikel einheitlichen Hilfsstofffraktion (beispielsweise 15-80 Mikrometer) bestehen oder gegebenenfalls ein Gemisch von gröberem Hilfsstoff mit einer mittleren Teilchengröße von 15 bis 80 Mikrometer und feinerem Hilfsstoff mit einer mittleren Teilchengröße von 1 bis 9 Mikrometer darstellen. Werden Hilfsstoffgemische aus gröberen und feineren Hilfsstoffanteilen eingesetzt, beträgt der Anteil von feinerem Hilfsstoff an der Gesamthilfsstoffmenge vorzugsweise 1 bis 20%.

Als physiologisch unbedenkliche Hilfsstoffe seien beispielsweise genannt Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

### Figuren

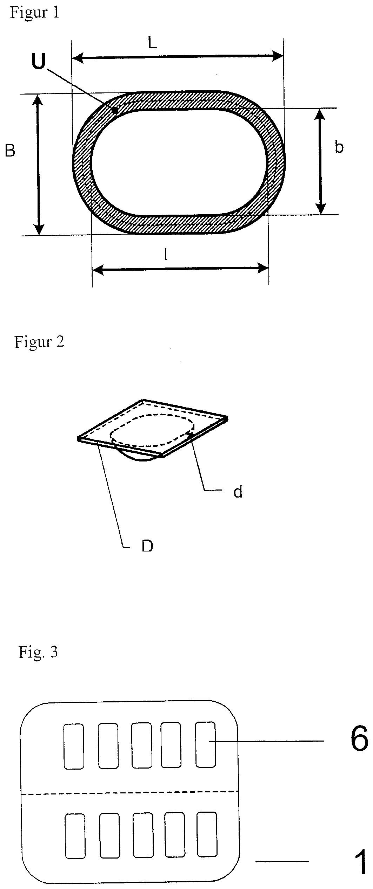

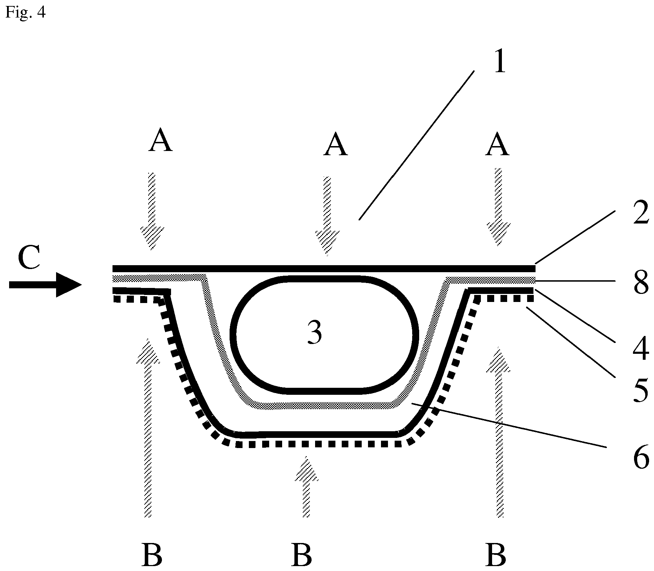

In den Figuren 1 bis 4 sind beispielhaft typische Blistervarianten dargestellt.

Fig. 1 zeigt ein Beispiel für eine Blistertasche mit den Siegelnahtabmaßen, die die mittlere Siegelnahtlänge U für Wasserdampf bilden. Der Blister ist in Draufsicht dargestellt. Die äußere Länge der Blistertasche L (von äußeren Ende zum äußeren Ende) beträgt beispielsweise 36,5 mm, die Länge der Kavität 1 beträgt 28,5 mm, die äußere Breite beträgt 25,5 mm, die Breite der Kavität beträgt 17,5mm. In anderen Ausführungsformen weist die Blisterkavität ein Volumen von 21 mm³ bei einer Fläche von 22,5 mm² auf, d.h. die Länge des Blisters L (von äußeren Ende zum äußeren Ende) beträgt ca. 13 mm, die Länge der Kavität 1 beträgt 4 mm, die äußere Breite beträgt 7,5 mm, die Breite der Kavität beträgt 2,65 mm.

Fig. 2 zeigt den Permeationsweg d und die Permeationsfläche (nach Außen orientierte Oberfläche der Siegellackschicht) D an.

In Figur 3. ist ein im Rahmen der vorliegenden Erfindung typischer Blister (1) mit mehreren Näpfen/Kavitäten (6) in Draufsicht dargestellt.

Fig. 4 zeigt einen Blister im Querschnitt, wobei nur ein Napf (6) dargestellt ist.
Der Blister besteht aus einer Siegelfolie (2), die über einer Tiefziehfolie mit mehreren nicht miteinander verbunden Näpfen (6) zur Aufnahme des pharmazeutischen Produktes (3) liegt, einer unteren Wannenschicht (4) und der Schutzschicht (5) unterhalb der Wannenschicht (4).
Die Pfeile A zeigen auf die Deckschicht (2) und sollen den Diffusionsweg von Feuchtigkeit durch die Deckschicht darstellen.

Die Pfeile B zeigen auf die bodenseitigen Schicht (8, 4, 5) und sollen den Diffusionsweg von Feuchtigkeit durch die Bodenschicht darstellen.
Der Pfeil C zeigt auf die Verbindungsstelle zwischen der Deck- und der Bodenschicht und den Weg, den Feuchtigkeit durch diesen Bereich des Blisters nehmen kann.

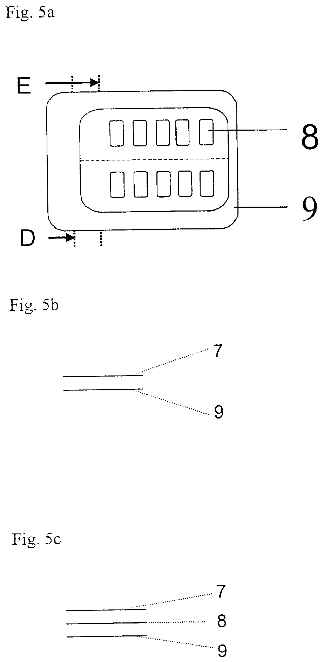

Fig. 5 zeigt eine Variante bestehend aus einer deckseitigen Siegelfolie (2, 7), einer bodenseitigen Aluminiumfolie (9) und einer dazwischen liegenden Kunststofffolie (8), die vollständig von den Aluminiumfolien (7) und (9) umgeben ist, dabei zeigt Fig. 5a die Draufsicht, wobei die Deckfolie, die genauso groß ist wie die Bodenfolie und darüber liegt, nicht dargestellt ist. Fig. 5b zeigt den Querschnitt entlang der Kante D und Fig. 5c zeigt den Querschnitt entlang der Kante E. Die Siegellackschicht ist nicht dargestellt.

Im Rahmen der vorliegenden Erfindung sind Blister mit folgender Schichtenabfolge bevorzugt:
Eine Siegelfolie (2) bestehend aus einer ersten Schicht (d.h. äußerste Schicht) aus Papier (20 bis 100 g/m²) oder Lack (0,5 bis 3 g/m²), eine darunter befindliche zweite Schicht aus Polyethylenterephthalat, bevorzugt in einer Dicke von 5 bis 20 Mikrometer, stärker bevorzugt 10 bis 15 Mikrometer, und schließlich eine Schicht aus Aluminiumfolie in einer bevorzugten Stärke von 10 bis 60 Mikrometer, bevorzugt 10 bis 50 Mikrometer und ganz bevorzugt 15 bis 40 Mikrometer.
Darunter ist das Basiselement zur Aufnahme des pharmazeutischen Produktes (3) angeordnet, die z.B. aus einer dreischichtigen Folie in einer bevorzugten Dicke von 30 bis 500 Mikrometer, besonders bevorzugt 60 bis 300 Mikrometer ausgebildet ist. Diese Folie besteht zunächst aus einer PVC-Schicht auf der produktberührenden Seite, deren Dicke bevorzugt 10 bis 200 Mikrometer, besonders bevorzugt 20 bis 70 Mikrometer beträgt dann aus einer Aluminiumschicht, deren Dicke bevorzugt 30 bis 60 Mikrometer, besonders bevorzugt 35 bis 50 Mikrometer beträgt. Diese Aluminiumschicht ist wiederum bedeckt von einer Schicht aus Polyamid mit einer bevorzugten Stärke von 10 bis 40 Mikrometer, besonders bevorzugt 20 bis 30 Mikrometer.
einer Schicht aus Polyamid mit einer bevorzugten Stärke von 10 bis 40 Mikrometer, besonders bevorzugt 20 bis 30 Mikrometer.
Dabei können einzelne Schichten, z.B. die Papierschicht weggelassen werden. Die erfindungsgemäße Heißsiegellackschicht wird der Einfachheit halber hier nicht berücksichtigt, sie befindet sich jedoch wieder zwischen dem Basiselement und der Siegelfolie.

Eine weitere Ausführungsform eines Blisters weist nachstehende Schichtenabfolge auf (von oben nach unten): Schutzlack, Druckfarbe, Schutzlack, 38 Mikrometer Siegelfolie (Aluminiumfolie), Heißsiegellackschicht, 30 Mikrometer Bodenfolie mit Kavität (PVC-Folie), Klebeschicht, 45 Mikrometer Aluminiumfolie, Haftvermittler, Klebeschicht, 25 Mikrometer Folie aus oPA (orientieres Polyamid).

Eine weitere Ausführungsform ist ein Blister aus drei Folien. Zunächst einer Deckfolie aus einer 38 Mikrometer dicken Deckschicht aus Aluminium, dann aus einer 250 Mikrometer starken Wannenfolie aus PVC zur Aufnahme des pharmazeutischen Produktes, und einer darunter liegenden Folie bestehend aus einer Schicht Aluminium in einer Stärke von 45 Mikrometern und einer bodenseitig abschließenden Schicht aus Polyamid in einer Stärke von 25 Mikrometer. Die erfindungsgemäße Siegellackschicht liegt zwischen dem Basiselement (in diesem Fall der Wannenfolie) und der darüber liegenden Siegelfolie aus Aluminium.

## Patentansprüche

1. Blister (1) zur Aufnahme einer oder mehrerer Einzeldosierungen einer Arzneimittelformulierung, bevorzugt einer Inhalationsformulierung, wobei jede Einzeldosierung in einer vollständig verschlossenen Kavität (6) vorliegt, enthaltend
a) ein Basiselement mit wenigstens einer nach wenigstens einer Seite hin offenen Kavität (6),
b) eine Siegelfolie (2), die die wenigstens eine Öffnung der wenigstens einen Kavität unter Ausbildung eines Hohlraums mit der darin eingebetteten Arzneimittelformulierung verschließt und
einer zwischen dem Basiselement und der Siegelfolie (2) liegende Siegellackschicht, wobei die Siegellackschicht eine Höhe hat die bis zu 5 Mikrometer beträgt, und wobei der Blister Kanten enthält, **dadurch gekennzeichnet dass** die nach Außen orientierten Kanten des Blisters umgefalzt sind.

2. Blister (1) nach Anspruch 1 **dadurch gekennzeichnet, dass** die Höhe der Siegellackschicht zwischen 1 und 4,5 Mikrometer, insbesondere bevorzugt zwischen 2 und 4,5 Mikrometer und am stärksten bevorzugt zwischen 2 und 4 Mikrometer liegt.

3. Blister (1) nach einem der vorangegangenen Ansprüche, **dadurch gekennzeichnet, dass** die Wasserdampfpermeabilität durch die Siegellackschicht im stationären Gleichgewichtszustand bei kleiner gleich 320 Mikrogramm / mm² Siegellackschicht, bevorzugt bei 60 bis 280 Mikrogramm / mm² Siegellackschicht, stärker bevorzugt bei 120 bis 280 Mikrogramm / mm² Siegellackschicht, stärker bevorzugt bei 120 bis 250 Mikrogramm / mm² Siegellackschicht liegt, gemessen bei einer relativen Luftfeuchtigkeit in der Kavität (6) von 16% und einer Luftfeuchtigkeit in der Außenumgebung des Blisters (1) von 75%, bei einer Temperatur von 40°C und über eine Zeitdauer von 6 Monaten.

4. Blister (1) nach einem der vorangegangen Ansprüche, **dadurch gekennzeichnet, dass** die Kavität (6) eine Länge von bis zu 10 mm, eine Breite von bis zu 10 mm aufweist.

5. Blister (1) nach einem der vorangegangen Ansprüche, **dadurch gekennzeichnet, dass** die Siegelfolie (2) so mit dem Basiselement verbunden ist, dass die Öffnung jeder Kavität (6) vollständig verschlossen ist, wobei eine geschlossene Siegelnaht die Öffnung einer jeden Kavität (6) umläuft.

6. Blister (1) nach einem der vorangegangen Ansprüche, **dadurch gekennzeichnet, dass** das Basiselement eine Aluminiumfolie (9) und die Siegelfolie (2) eine Aluminiumfolie (7) ist.

7. Blister (1) nach einem der vorangegangen Ansprüche, **dadurch gekennzeichnet, dass** das die Siegellackschicht ein Polyacrylat und/oder ein Polyethylen ist.

8. Blister (1) nach einem der vorangegangen Ansprüche, **dadurch gekennzeichnet, dass** die Menge an aufgetragenem Siegellack vor dem Versiegelungsvorgang bis zu 10 g pro Quadratmeter beträgt, bevorzugt 1 bis 7 g pro Quadratmeter, besonders bevorzugt 2 bis 5,5 g pro Quadratmeter.

9. Blister (1) nach einem der vorangegangen Ansprüche, **dadurch gekennzeichnet, dass** die nach Außen orientierten Kanten des Blisters (1) mit einer Folie versiegelt ist.

10. Blister (1) nach einem der Ansprüche 1 bis 9, **dadurch gekennzeichnet dass** der Blister (1) eine einzige Kavität (6) zur Aufnahme einer einzigen Einzeldosierung, eine einzige Kavität (6) zur Aufnahme von mehreren separat verpackten Einzeldosierungen, ein Band mit einer Reihe linear angeordneter Kavitäten (6), ein Band mit wenigstens zwei Reihen linear angeordneter Kavitäten (6) oder eine Blisterscheibe ist.

11. Blister (1) nach einem der Ansprüche 1 bis 10 **dadurch gekennzeichnet, dass** das Basiselement von einer Aluminiumfolie (9) mit einer Kunststoffbeschichtung gebildet wird, wobei als Material für die Kunststoffbeschichtung Polyvinylchlorid (PVC), Cycloolefin-Copolymer (COC), Cycloolefinpolymer (COP), Polychlorotrifluorethylen, Polyethylen, Polypropylen, Polyvinylidenchloride (PVDC), Polyethylenterephthalalt, Polycarbonate, Polyester, Polyacrylate, Polyamide oder ein anderer Kunststoff verwendet wird, insbesondere wobei sich die von dem Kunststoff gebildete Kunststoffbeschichtung auf der die Kavität (6) aufweisenden Seite des Blisters (1) befindet.

12. Blister (1) nach einem der Ansprüche 1 bis 10, **dadurch gekennzeichnet dass** der Blister (1) eine Schichtabfolge von Aluminium, Kunststoff, Siegellack, Aluminium aufweist, wobei als Kunststoff ein Kunststoff ausgewählt aus der Gruppe PVC, COC, COP, Polychlorotrifluorethylen, Polyethylen, Polypropylen, PVDC, Polyethylenterephthalalt, Polycarbonat, Polyester, Polyacrylat, Polyamid, bevorzugt PVC, verwendet wird und/oder die Höhe der Kunststoffschicht 50 Mikrometer oder weniger, bevorzugt 40 Mikrometer oder weniger, stärker bevorzugt 30 Mikrometer oder weniger beträgt.

13. Blister (1) nach einem der Ansprüche 1 bis 10, **dadurch gekennzeichnet, dass** das Basiselement aus einer dreischichtigen Folie aus einer PVC-Schicht an der produktberührenden Seite, einer Aluminiumschicht und einer Polyamidschicht ausgebildet ist.

14. Blister (1) nach Anspruch 13, **dadurch gekennzeichnet, dass** die Dicke der PVC-Schicht 10-200 Mikrometer, besonders bevorzugt 20 bis 70 Mikrometer beträgt.

15. Inhalator enthaltend einen Blister (1) nach einem der Ansprüche 1 bis 14.

## Claims

1. Blister (1) for accommodating one or more individual doses of a drug formulation, preferably an inhalation formulation, each individual dose being present in a completely sealed cavity (6), containing
a) a base element having at least one cavity (6) open to at least one side,
b) a sealing film (2) that seals the at least one opening of the at least one cavity to form a hollow space having the drug formulation embedded therein, and
a sealing lacquer layer lying between the base element and the sealing film (2), the sealing lacquer layer having a height of up to 5 micrometres, and the blister containing edges, **characterised in that** the outwardly oriented edges of the blister are folded over.

2. Blister (1) according to claim 1, **characterised in that** the height of the sealing lacquer layer is between 1 and 4.5 micrometres, particularly preferably between 2 and 4.5 micrometres and most preferably between 2 and 4 micrometres.

3. Blister (1) according to either of the preceding claims, **characterised in that** the water vapour permeability through the sealing lacquer layer in the state of static equilibrium is less than or equal to 320 micrograms/mm² sealing lacquer layer, preferably is 60 to 280 micrograms/mm² sealing lacquer layer, more preferably is 120 to 280 micrograms/mm² sealing lacquer layer, and even more preferably is 120 to 250 micrograms/mm² sealing lacquer layer, measured at a relative atmospheric humidity in the cavity (6) of 16% and an atmospheric humidity in the external surroundings of the blister (1) of 75%, at a temperature of 40°C and over a period of 6 months.

4. Blister (1) according to any of the preceding claims, **characterised in that** the cavity (6) has a length of up to 10 mm and a width of up to 10 mm.

5. Blister (1) according to any of the preceding claims, **characterised in that** the sealing film (2) is connected to the base element such that the opening of each cavity (6) is completely sealed, a closed sealing seam surrounding the opening of every cavity (6).

6. Blister (1) according to any of the preceding claims, **characterised in that** the base element is an aluminium film (9) and the sealing film (2) is an aluminium film (7).

7. Blister (1) according to any of the preceding claims, **characterised in that** the sealing lacquer layer is a polyacrylate and/or a polyethylene.

8. Blister (1) according to any of the preceding claims, **characterised in that** the amount of applied sealing lacquer before the sealing process is up to 10 g per square metre, preferably 1 to 7 g per square metre, particularly preferably 2 to 5.5 g per square metre.

9. Blister (1) according to any of the preceding claims, **characterised in that** the outwardly oriented edges of the blister (1) are sealed by means of a film.

10. Blister (1) according to any of claims 1 to 9, **characterised in that** the blister (1) is a single cavity (6) for accommodating a single individual dose, a single cavity (6) for accommodating a plurality of separately packaged individual doses, a strip having a row of linearly arranged cavities (6), a strip having at least two rows of linearly arranged cavities (6), or a blister pack.

11. Blister (1) according to any of claims 1 to 10, **characterised in that** the base element is formed by an aluminium film (9) having a plastics coating, as a material for the plastics coating polyvinyl chloride (PVC), cycloolefin copolymer (COC), cycloolefin polymer (COP), polychlorotrifluoroethylene, polyethylene, polypropylene, polyvinylidene chlorides (PVDC), polyethylene terephthalate, polycarbonates, polyesters, polyacrylates, polyamides or another plastics material being used, in particular the plastics coating formed by the plastics material being located on the side of the blister (1) that comprises the cavity (6).

12. Blister (1) according to any of claims 1 to 10, **characterised in that** the blister (1) comprises a layer sequence of aluminium, plastics material, sealing lacquer, aluminium, wherein as the plastics material a plastics material selected from the group PVC, COC, COP, polychlorotrifluoroethylene, polyethylene, polypropylene, PVDC, polyethylene terephthalate, polycarbonate, polyester, polyacrylate, polyamide, preferably PVC, is used and/or the height of the plastics layer is 50 micrometres or less, preferably 40 micrometres or less, more preferably 30 micrometres or less.

13. Blister (1) according to any of claims 1 to 10, **characterised in that** the base element is formed of a three-layered film made of a PVC layer on the side in contact with the product, an aluminium layer and a polyamide layer.

14. Blister (1) according to claim 13, **characterised in that** the thickness of the PVC layer is 10-200 micrometres, particularly preferably 20 to 70 micrometres.

15. Inhaler containing a blister (1) according to any of claims 1 to 14.

## Revendications

1. Blister (1) pour la réception d'une ou plusieurs doses individuelles d'une formulation de médicament, de préférence d'une formulation d'inhalation, dans lequel chaque dose individuelle est disponible dans une cavité entièrement fermée (6), contenant
a) un élément de base avec au moins une cavité (6) ouverte vers au moins un côté,
b) un film de scellage (2), qui ferme l'au moins une ouverture de l'au moins une cavité en formant un espace creux avec la formulation de médicament qui y est incorporée et
une couche de vernis de scellage située entre l'élément de base et le film de scellage (2), dans lequel la couche de vernis de scellage a une hauteur allant jusqu'à 5 micromètres, et dans lequel le blister contient des arêtes, **caractérisé en ce que** les arêtes du blister orientées vers l'extérieur sont repliées.

2. Blister (1) selon la revendication 1 **caractérisé en ce que** la hauteur de la couche de vernis de scellage est entre 1 et 4,5 micromètres, en particulier de préférence entre 2 et 4,5 micromètres et de manière préférée entre toutes entre 2 et 4 micromètres.

3. Blister (1) selon l'une quelconque des revendications précédentes **caractérisé en ce que** la perméabilité à la vapeur d'eau par la couche de vernis de scellage à l'état d'équilibre stationnaire est inférieure ou égale à 320 microgrammes/mm² de couche de vernis de scellage, de préférence de 60 à 280 microgrammes/mm² de couche de vernis de scellage, de manière davantage préférée de 120 à 280 microgrammes/mm² de couche de vernis de scellage, de manière davantage préférée de 120 à 250 microgrammes/mm² de couche de vernis de scellage, mesurée pour une humidité relative de l'air dans la cavité (6) de 16 % et une humidité de l'air dans l'environnement extérieur du blister (1) de 75 %, pour une température de 40 °C et sur une période de 6 mois.

4. Blister (1) selon l'une quelconque des revendications précédentes, **caractérisé en ce que** la cavité (6) présente une longueur allant jusqu'à 10 mm, une largeur allant jusqu'à 10 mm.

5. Blister (1) selon l'une quelconque des revendications précédentes, **caractérisé en ce que** le film de scellage (2) est relié à l'élément de base de sorte que l'ouverture de chaque cavité (6) est entièrement fermée, dans lequel un joint de scellage fermé entoure l'ouverture de chaque cavité (6).

6. Blister (1) selon l'une quelconque des revendications précédentes, **caractérisé en ce que** l'élément de base est une feuille d'aluminium (9) et le film de scellage (2) est une feuille d'aluminium (7).

7. Blister (1) selon l'une quelconque des revendications précédentes, **caractérisé en ce que** la couche de vernis de scellage est un polyacrylate et/ou un polyéthylène.

8. Blister (1) selon l'une quelconque des revendications précédentes, **caractérisé en ce que** la quantité de vernis de scellage appliquée avant l'opération de scellage va jusqu'à 10 g par mètre carré, de préférence est de 1 à 7 g par mètre carré, de manière particulièrement préférée 2 à 5,5 g par mètre carré.

9. Blister (1) selon l'une quelconque des revendications précédentes, **caractérisé en ce que** les arêtes du blister (1) orientées vers l'extérieur sont scellées avec un film.

10. Blister (1) selon l'une quelconque des revendications 1 à 9, **caractérisé en ce que** le blister (1) est une seule cavité (6) pour la réception d'une seule dose individuelle, une seule cavité (6) pour la réception de plusieurs doses individuelles conditionnées séparément, une bande avec une série de cavités (6) agencées de façon linéaire, une bande avec au moins deux rangées de cavités (6) agencées de façon linéaire ou un disque de blister.

11. Blister (1) selon l'une quelconque des revendications 1 à 10, **caractérisé en ce que** l'élément de base est formé par une feuille d'aluminium (9) avec un revêtement plastique, dans lequel du polychlorure de vinyle (PVC), du copolymère cyclo-oléfine (COC), du polymère cyclo-oléfine (COP), du polychlorotrifluoroéthylène, du polyéthylène, du polypropylène, du polychlorure de vinylidène (PVDC), du polytéréphtalate d'éthylène, du polycarbonate, du polyester, du polyacrylate, du polyamide ou un autre plastique est utilisé en tant que matériau pour le revêtement plastique, en particulier dans lequel le revêtement plastique formé par le plastique se trouve sur le côté du blister (1) présentant la cavité (6).

12. Blister (1) selon l'une quelconque des revendications 1 à 10, **caractérisé en ce que** le blister (1) présente une séquence de couches d'aluminium, de plastique, de vernis de scellage, d'aluminium, dans lequel un plastique est choisi en tant que plastique dans le groupe PVC, COC, COP, polychlorotrifluoroéthylène, polyéthylène, polypropylène, PVDC, polytéréphtalate d'éthylène, polycarbonate, polyester, polyacrylate, polyamide, de préférence du PVC, et/ou la hauteur de la couche plastique est de 50 micromètres ou moins, de préférence de 40 micromètres ou moins, de manière davantage préférée de 30 micromètres ou moins.

13. Blister (1) selon l'une quelconque des revendications 1 à 10, **caractérisé en ce que** l'élément de base est réalisé en un film à trois couches en une couche de PVC au niveau du côté en contact avec le produit, une couche d'aluminium et une couche de polyamide.

14. Blister (1) selon la revendication 13, **caractérisé en ce que** l'épaisseur de la couche de PVC est de 10-200 micromètres, de manière particulièrement préférée de 20 à 70 micromètres.

15. Inhalateur contenant un blister (1) selon l'une quelconque des revendications 1 à 14.
